# EUROPEAN PATENT APPLICATION

(11) **EP 4 533 999 A1**
(43) Date of publication of application: **09.04.2025**
(21) Application number: 24200282.2
(22) Date of filing: 13.09.2024
(51) Int. Cl.: A61B 3/00, A61B 3/103

(54) **PHOTOREFRACTION VISION SCREENING DEVICE**

(30) Priority: 05.10.2023 KR 20230132363
(71) Applicant: Huvitz Co., Ltd., Anyang-si, Gyeonggi-do 14055 (KR)
(72) Inventor: LEE, Jae Hyuk, 14055 Anyang-si (KR); KIM, Kang Min, 14055 Anyang-si (KR); JO, Sunhyung, 14055 Anyang-si (KR); LIM, Jong Min, 14055 Anyang-si (KR); LEE, Won Chan, 14055 Anyang-si (KR); KIM, Tae Kyun, 14055 Anyang-si (KR); SONG, In Seok, 14055 Anyang-si (KR)
(74) Representative: Henkel & Partner mbB

(57) **Abstract**

A photorefraction vision screening device is disclosed for examining visual diseases by performing pupil detection and refraction test of the eye. The photorefraction vision screening device (100) may include: a measurement light irradiator (50) configured to irradiate measurement light to an eye to be examined; a measurement light controller (60) configured to control light-emitting elements of the measurement light irradiator (50); an image detector (20) configured to detect an image formed on the retina of the eye to be examined by the measurement light having been irradiated from the light-emitting elements of the measurement light irradiator (50) and having passed through the pupil of the eye to be examined; and a calculation part (30) configured to calculate refractive power of the eye to be examined based on the image of the measurement light detected by the image detector (20), wherein two or more measurement light sources (50a, 50b, 50c, 50d, 50e, 50f) may be arranged radially with the image detector (20) as the center in the measurement light irradiator (50), and each of the measurement light sources (50a, 50b, 50c, 50d, 50e, 50f) includes three or more light-emitting elements (51, 52, 53, 54) sequentially arranged in one direction, and wherein the measurement light controller (60) may be configured to select two or more of the light-emitting elements (51, 52, 53, 54) sequentially arranged in each of the measurement light sources (50a, 50b, 50c, 50d, 50e, 50f) and turn on the selected ones simultaneously, and the number of light-emitting elements that are simultaneously turned on is less than a total number of light-emitting elements (51, 52, 53, 54).

## Description

### CROSS-REFERENCE TO RELATED APPLICATION

This application claims benefit of priority to Korean Patent Application No. 10-2023-0132363 filed on October 05, 2023, the entire contents of which are incorporated herein by reference.

### FIELD OF THE DISCLOSURE

The present disclosure relates to a photorefraction vision screening device and, more particularly, to a photorefraction vision screening device for performing pupil detection and refraction test of an eye to examine visual diseases.

### BACKGROUND

A photorefraction vision screening device is a device that detects and analyzes the eye's response to light, and is commonly referred to as a "vision screener". When external measurement light enters the eye through the pupil and crystalline lens and is focused on a point on the retina, the focused light is reflected from the point on the retina and then emitted out of the eye through the pupil. Since the pattern of light emitted through the pupil is determined by the optical system and refraction abnormalities of the eye, the presence or absence of a visual disease in the eye may be examined by detecting the pattern of light emitted through the pupil.

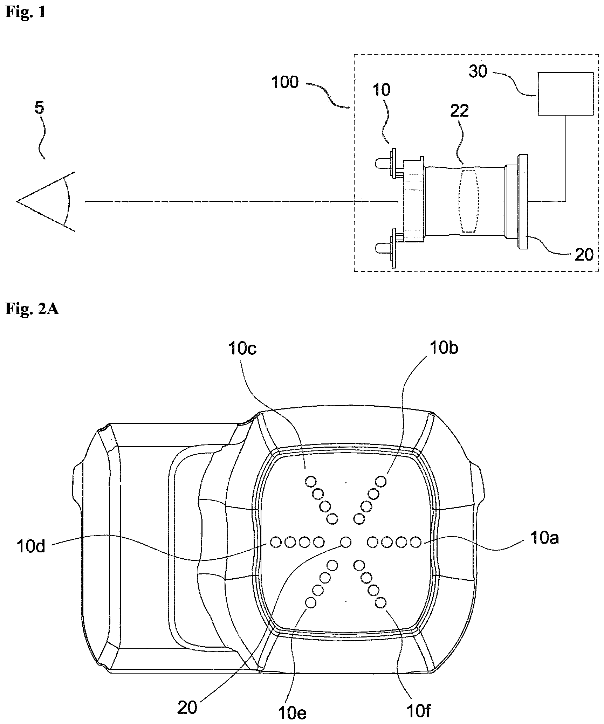

FIG. 1 is a diagram showing a vision test process using a typical photorefraction vision screening device. As shown in FIG. 1, the typical photorefraction vision screening device 100 includes a measurement light source 10 that irradiates measurement light to the eye to be examined 5, an image detector 20 that detects an image formed on the retina by the measurement light having been irradiated from the measurement light source 10 and having passed through the pupil of the eye to be examined 5, and a calculation part 30 that calculates the refractive power of the eye to be examined 5 from the image of the measurement light detected by the image detector 20. In FIG. 1, reference numeral 22 denotes a camera lens that focuses an image of the measurement light formed on the retina.

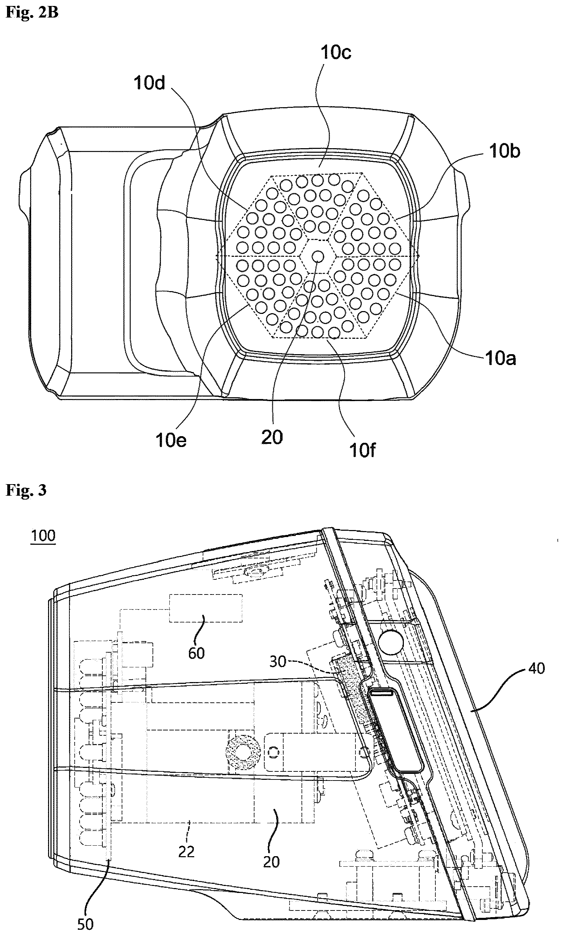

FIGS. 2A and 2B are diagrams showing various forms of the measurement light source 10 in a typical photorefraction vision screening device 100. In the case where the eye to be examined (5) is astigmatic, the refractive power of the eye to be examined 5 is different in every direction (e.g., up, down, left, right) of the eye to be examined 5. So, as shown in FIGS. 2A and 2B, for the measurement light source 10 to irradiate measurement light in every direction (specifically, six directions) of the eye to be examined 5, a number of measurement light sources 10a, 10b, 10c, 10d, 10e and 10f are arranged radially around the image detector 20.

In the example shown in FIG. 2A, each of the measurement light sources 10a, 10b, 10c, 10d, 10e and 10f positioned in every direction includes four light-emitting elements, specifically LEDs, and the four LEDs positioned at different heights in one direction are turned on in sequence to generate measurement light. In this case, the image detector 20 and the calculation part 30 analyze a total of 24 measurement light images to measure the vision of the eye to be examined 5. In the example shown in FIG. 2B, each of the measurement light sources 10a, 10b, 10c, 10d, 10e and 10f positioned in every direction includes 14 LEDs, and the 14 LEDs positioned in one direction are simultaneously turned on to generate measurement light. In this case, the image detector 20 and the calculation part 30 analyze a total of six measurement light images to measure the vision of the eye to be examined 5.

As shown in FIG. 2A, the method of measuring the vision of the eye to be examined 5 by irradiating measurement light using a single LED and detecting the form in which the measurement light is reflected within the pupil is relatively accurate, but the intensity of the measurement light is weak and it is easily affected by external light. Meanwhile, as shown in FIG. 2B, the method of irradiating measurement light by turning on multiple LEDs simultaneously is less affected by external light, but has the disadvantage of low examination accuracy.

### [Prior Art Documents]

### [Patent Documents]

(Patent document 1) US Patent No. 9,408,535
(Patent document 2) US Patent No. 9,402,538
(Patent document 3) US Patent No. 9,237,846
(Patent document 4) International Patent Publication No. WO 2021/164864

### SUMMARY OF THE DISCLOSURE

### TECHNICAL OBJECTS

An object of the present disclosure is to provide a photorefraction vision screening device that is capable of accurately and precisely examining the eye to be examined with less influence of external light by increasing the intensity of measurement light emitted from the measurement light source without increasing the number of light-emitting elements included in the measurement light source.

Another object of the present disclosure is to provide a photorefraction vision screening device that is capable of adjusting the angle between the measurement light irradiated onto the eye to be examined and the eye to be examined by adjusting the position of light-emitting elements irradiating measurement light.

### TECHNICAL SOLUTION

To achieve the above objectives, the present disclosure provides a photorefraction vision screening device (100), which may include: a measurement light irradiator (50) configured to irradiate measurement light to an eye to be examined; a measurement light controller (60) configured to control light-emitting elements of the measurement light irradiator (50); an image detector (20) configured to detect an image formed on the retina of the eye to be examined by the measurement light having been irradiated from the light-emitting elements of the measurement light irradiator (50) and having passed through the pupil of the eye to be examined; and a calculation part (30) configured to calculate refractive power of the eye to be examined based on the image of the measurement light detected by the image detector (20), wherein two or more measurement light sources (50a, 50b, 50c, 50d, 50e, 50f) may be arranged radially with the image detector (20) as the center in the measurement light irradiator (50), and each of the measurement light sources (50a, 50b, 50c, 50d, 50e, 50f) includes three or more light-emitting elements (51, 52, 53, 54) sequentially arranged in one direction, and wherein the measurement light controller (60) may be configured to select two or more of the light-emitting elements (51, 52, 53, 54) sequentially arranged in each of the measurement light sources (50a, 50b, 50c, 50d, 50e, 50f) and turn on the selected ones simultaneously, and the number of light-emitting elements that are simultaneously turned on is less than a total number of light-emitting elements (51, 52, 53, 54).

### EFFECTS OF THE DISCLOSURE

The photorefraction vision screening device according to the present disclosure may increase the intensity of measurement light emitted from the measurement light source while reducing the influence of external light to thereby enable accurate and detailed examination of the eye to be examined. In addition, the photorefraction vision screening device according to the present disclosure may adjust the angle between the measurement light irradiated onto the eye to be examined and the eye to be examined by adjusting the position of the light-emitting elements irradiating measurement light.

### BRIEF DESCRIPTION OF THE DRAWINGS

FIG. 1 is a diagram showing a vision test process using a typical photorefraction vision screening device.
FIGS. 2A and 2B are diagrams showing various forms of a measurement light source 10 in a typical photorefraction vision screening device 100.
FIG. 3 is a side view of a photorefraction vision screening device according to an embodiment of the present disclosure.
FIG. 4 is a front view of a measurement light irradiator 50 used in the photorefraction vision screening device according to an embodiment of the present disclosure.
FIG. 5 is a diagram for depicting a method of irradiating measurement light in the photorefraction vision screening device according to an embodiment of the present disclosure.

### DETAILED DESCRIPTION OF THE PREFERRED EMBODIMENTS

Hereinafter, the present disclosure will be described in detail with reference to the accompanying drawings. In the description of the present disclosure, detailed descriptions of related well-known general functions or configurations are omitted. Additionally, in the drawings, the same reference symbols are given to components that are identical or similar to those in the related art.

FIG. 3 is a side view of a photorefraction vision screening device according to an embodiment of the present disclosure, where internal structures are indicated by dotted lines for the purpose of explanation. As shown in FIG. 3, the photorefraction vision screening device 100 according to the present disclosure includes a measurement light irradiator 50 that irradiates measuring light to the eye to be examined, a measurement light controller 60 that controls light-emitting elements of the measurement light irradiator 50, an image detector 20 that detects an image formed on the retina by the measurement light having been irradiated from the light-emitting elements of the measurement light irradiator 50 and having passed through the pupil of the eye to be examined, and a calculation part 30 that calculates the refractive power of the eye to be examined from the image of the measurement light detected by the image detector 20. If necessary, a display unit 40, which displays the image of the measurement light detected by the image detector 20 and the refractive power of the eye to be examined calculated by the calculation part 30, is mounted on the rear side of the photorefraction vision screening device 100. Additionally, a typical camera lens 22 that focuses an image of the measurement light formed on the retina may be mounted on the front end of the image detector 20.

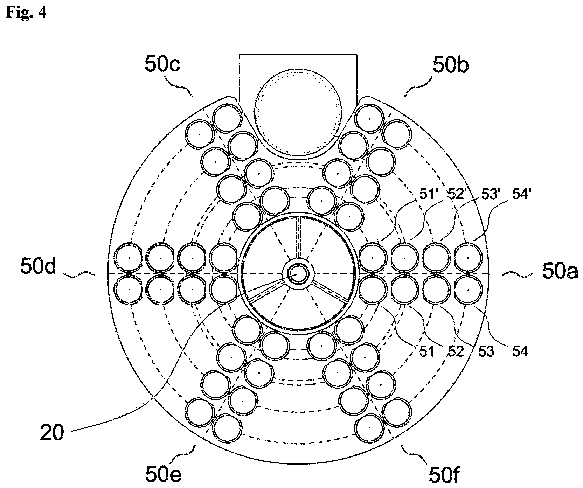

FIG. 4 is a front view of the measurement light irradiator 50 used in the photorefraction vision screening device according to an embodiment of the present disclosure. As shown in FIG. 4, in the measurement light irradiator 50 used in the present disclosure, to irradiate measurement light to the eye to be examined in two or more directions, preferably in three to eight directions, particularly preferably in six directions, two or more, preferably three to eight, particularly preferably six measurement light sources 50a, 50b, 50c, 50d, 50e and 50f are arranged radially at equal angular intervals around the image detector 20 (with the image detector 20 being the center). For example, as shown in FIG. 4, if the measurement light irradiator 50 includes six measurement light sources 50a, 50b, 50c, 50d, 50e and 50f, they are arranged radially at equal angular intervals of 60 degrees.

Here, each of the measurement light sources 50a, 50b, 50c, 50d, 50e and 50f includes three or more, preferably four to eight, more preferably four to six, for example four light-emitting elements 51, 52, 53 and 54 arranged sequentially in one direction. Preferably, the light-emitting elements 51, 52, 53 and 54 are arranged radially at equal intervals from the image detector 20 positioned at the center. Additionally, in each of the measurement light sources 50a, 50b, 50c, 50d, 50e and 50f, the light-emitting elements 51, 52, 53 and 54 may be arranged in two or more rows. For example, as shown in FIG. 4, each of the measurement light sources 50a, 50b, 50c, 50d, 50e and 50f may include first-row light-emitting elements 51, 52, 53 and 54 arranged sequentially in one direction, and second-row light-emitting elements 51', 52', 53' and 54' arranged side by side adjacent to the first-row light-emitting elements 51, 52, 53 and 54.

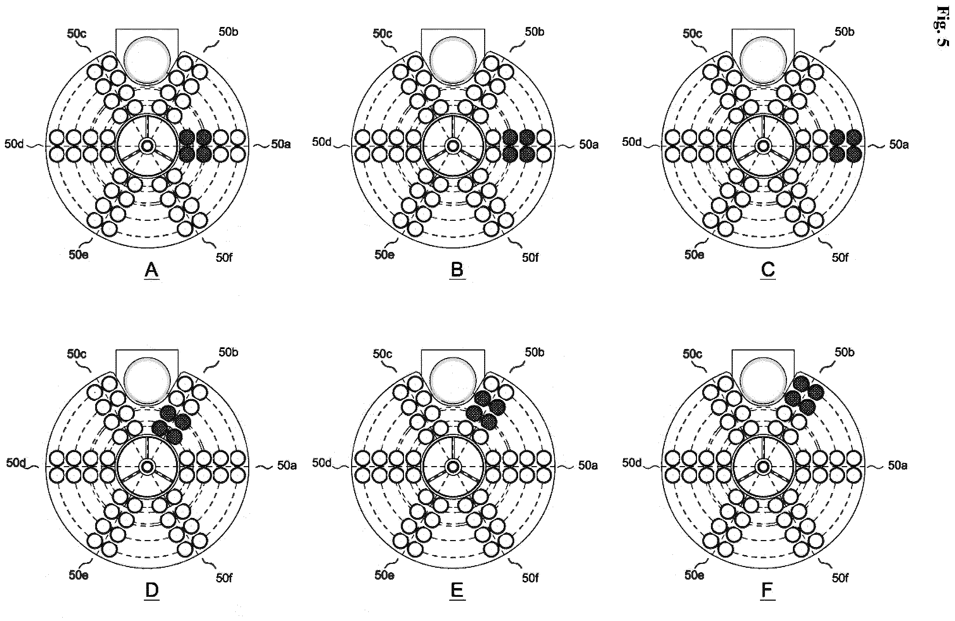

The measurement light controller 60 selects (combines) two or more, preferably two to four, for example two, among the light-emitting elements 51, 52, 53 and 54 sequentially arranged in each of the measurement light sources 50a, 50b, 50c, 50d, 50e and 50f and turns them on simultaneously. Here, the number of light-emitting elements that are simultaneously turned on is less than the total number of light-emitting elements 51, 52, 53 and 54. Preferably, the measurement light controller 60 causes two or more light-emitting elements that are consecutively positioned to emit light among the sequentially arranged light-emitting elements 51, 52, 53 and 54. For example, the first light-emitting element 51 and the second light-emitting element 52 are selected and turned on for the first time, then the second light-emitting element 52 and the third light-emitting element 53 are selected and turned on for the second time, and then the third light-emitting element 53 and the fourth light-emitting element 54 are selected and turned on for the third time. In this way, when two of the light-emitting elements 51, 52, 53 and 54 are simultaneously turned on, the lighting position of the measurement light sources 50a, 50b, 50c, 50d, 50e and 50f becomes the middle position of the two light-emitting elements that are turned on. In addition, when the process of turning on two or more light-emitting elements positioned consecutively is performed in sequence (A -> B -> C in FIG. 5), the light-emitting elements that are turned on in sequence (e.g., first-time turning-on and second-time turning-on) include at least one common light-emitting element (e.g., second light-emitting element 52). Hence, depending on the combination of the selected ones among the light-emitting elements 51, 52, 53 and 54, the light-emitting elements 51, 52, 53 and 54 positioned at different heights (corresponding to the distance from the image detector 20, or the distance from the pupil of the eye to be examined) in one direction emit measurement light, and the lighting position of the measurement light sources 50a, 50b, 50c, 50d, 50e and 50f changes. Therefore, the position of the measurement light irradiated to the eye to be examined is moved away from or closer to the eye to be examined. Here, it is preferable that the light emission of the light-emitting elements 51, 52, 53 and 54) is sequentially performed so that the position of the measurement light irradiated to the eye to be examined is moved away from or closer to the center of the eye to be examined.

FIG. 5 is a diagram for explaining a method of irradiating measuring light in the photorefraction vision screening device according to an embodiment of the present disclosure, which depicts a case where each of the measurement light sources 50a, 50b, 50c, 50d, 50e and 50f includes two rows of four light-emitting elements sequentially arranged in one direction, that is, first-row light-emitting elements 51, 52, 53 and 54 and second-row light-emitting elements 51', 52', 53' and 54', and the measurement light controller 60 turns on two of the first-row light-emitting elements 51, 52, 53 and 54 and two of the second-row light-emitting elements 51', 52', 53' and 54' in a sequential manner. In FIG. 5, the turned-on light-emitting elements are distinguished by hatching. As shown in FIG. 5, the measurement light controller 60 turns on the first and second light-emitting elements 51 and 52) of the first row and the first and second light-emitting elements 51' and 52' of the second row to thereby measure the vision of the eye to be examined at a first height (distance from the image detector 20, i.e., distance from the pupil of the eye to be examined) (A in FIG. 5). Next, the measurement light controller 60 turns on the second and third light-emitting elements 52 and 53 of the first row and the second and third light-emitting elements 52' and 53' of the second row to thereby measure the vision of the eye to be examined at a second height (distance from the image detector 20, i.e., distance from the pupil of the eye to be examined) (B in FIG. 5). Next, the measurement light controller 60 turns on the third and fourth light-emitting elements 53 and 54 of the first row and the third and fourth light-emitting elements 53' and 54' of the second row to thereby measure the vision of the eye to be examined at a third height (distance from the image detector 20, i.e., distance from the pupil of the eye to be examined) (C in FIG. 5). In this way, by measuring the vision of the eye to be examined at different heights in one direction, that is, in the direction of the first measurement light source 50a, the accuracy of vision measurement can be improved. When measurement light is irradiated to the eye to be examined at different heights, the angle at which the measurement light is irradiated to the eye to be examined changes, so the refractive power of the eye to be examined may be obtained under different conditions. Therefore, the reliability of refractive power measurement may be improved compared to the case where the refractive power is measured under only one condition (i.e., height).

Next, by repeating the same measurement process in a second direction, that is, in the direction of the second measurement light source 50b, the vision, specifically the refractive power, in that direction is measured (D, E, F in FIG. 5). By performing these measurements in third to sixth directions, that is, in the directions of the third to sixth measurement light sources 50c, 50d, 50e and 50f, the vision of the eye to be examined may be measured in six directions, making it possible to test whether the eye to be examined has astigmatism.

In the example shown in FIG. 5, four of the first-row light-emitting elements 51, 52, 53 and 54 and second-row light-emitting elements 51', 52', 53' and 54' are turned on three times in an overlapping manner in one direction (A, B, C in FIG. 5), and this process is performed in six directions. So, a total of 18 pupil images are obtained, and the vision of the eye to be examined may be examined by analyzing these images.

According to the present disclosure, by turning on and off the light-emitting elements 51, 52, 53, 54, 51', 52', 53' and 54' of the measurement light irradiator 50 in an overlapping manner, the intensity of the measurement light may be increased and the position where the measurement light is generated may be adjusted. Therefore, according to the present disclosure, detailed examination of the eye to be examined for myopia, astigmatism, strabismus, or the like is possible with less influence of external light.

Although the present disclosure has been described with reference to the attached drawings and exemplary embodiments, the present disclosure is not limited to the contents illustrated in the drawings and the embodiments described above. Although reference symbols are used in the claims below to aid understanding, the scope of the claims is not limited by those reference symbols and the contents depicted in the drawings, but should be interpreted comprehensively to include all modifications of the exemplary embodiments, and equivalent structures and functions.

## Claims

1. A photorefraction vision screening device (100) comprising:
a measurement light irradiator (50) configured to irradiate measurement light to an eye to be examined;
a measurement light controller (60) configured to control light-emitting elements of the measurement light irradiator (50);
an image detector (20) configured to detect an image formed on a retina of the eye to be examined by the measurement light having been irradiated from the light-emitting elements of the measurement light irradiator (50) and having passed through a pupil of the eye to be examined; and
a calculation part (30) configured to calculate refractive power of the eye to be examined based on the image of the measurement light detected by the image detector (20),
wherein two or more measurement light sources (50a, 50b, 50c, 50d, 50e, 50f) are arranged radially with the image detector (20) as a center in the measurement light irradiator (50), and each of the measurement light sources (50a, 50b, 50c, 50d, 50e, 50f) includes three or more light-emitting elements (51, 52, 53, 54) sequentially arranged in one direction,
wherein the measurement light controller (60) is configured to select two or more of the light-emitting elements (51, 52, 53, 54) sequentially arranged in each of the measurement light sources (50a, 50b, 50c, 50d, 50e, 50f) and turn on the selected ones simultaneously, and a number of light-emitting elements that are simultaneously turned on is less than a total number of light-emitting elements (51, 52, 53, 54).

2. The photorefraction vision screening device (100) of claim 1, wherein the measurement light controller (60) is configured to cause consecutively positioned two or more light-emitting elements among the sequentially arranged light-emitting elements (51, 52, 53, 54) to emit light simultaneously.

3. The photorefraction vision screening device (100) of claim 2, wherein light emission of the light-emitting elements (51, 52, 53, 54) is performed in sequence in a manner that a position of the measurement light being irradiated to the eye to be examined is moved away from or closer to a center of the eye to be examined.

4. The photorefraction vision screening device (100) of claim 1, wherein the light-emitting elements (51, 52, 53, 54) are arranged radially at equal intervals from the image detector (20) positioned at a center.

5. The photorefraction vision screening device (100) of claim 1, wherein each of the measurement light sources (50a, 50b, 50c, 50d, 50e, 50f) includes first-row light-emitting elements (51, 52, 53, 54) arranged sequentially in one direction, and second-row light-emitting elements (51', 52', 53', 54') arranged side by side adjacent to the first-row light-emitting elements (51, 52, 53, 54).

6. The photorefraction vision screening device (100) of claim 5, wherein six measurement light sources (50a, 50b, 50c, 50d, 50e, 50f) are arranged radially at equal angular intervals in the measurement light irradiator (50).

7. The photorefraction vision screening device (100) of claim 6, wherein the measurement light controller (60) is configured to measure the vision of the eye to be examined at a first height by turning on first and second light-emitting elements (51, 52) of the first row and first and second light-emitting elements (51', 52') of the second row, then measure the vision of the eye to be examined at a second height by turning on second and third light-emitting elements (52, 53) of the first row and second and third light-emitting elements (52', 53') of the second row, and then measure the vision of the eye to be examined at a third height by turning on third and fourth light-emitting elements (53, 54) of the first row and third and fourth light-emitting elements (53', 54') of the second row.
